# EUROPEAN PATENT APPLICATION

(11) **EP 3 461 476 A1**
(43) Date of publication of application: **03.04.2019**
(21) Application number: 17726123.7
(22) Date of filing: 05.05.2017
(51) Int. Cl.: A61K 9/28, A61K 9/48, A61K 9/20, A61K 31/4458, A61P 25/00

(54) **DELAYED-RELEASE TABLETS OF METHYLPHENIDATE**

(30) Priority: 05.05.2016 ES 201630582
(71) Applicant: Products & Technology, S.L., 08755 Castellbisbal Barcelona (ES)
(72) Inventor: FÁBREGAS VIDAL, José Luís, 08015 Barcelona (ES); RUIZ XIVILLÉ, Núria, 08912 Badalona Barcelona (ES)
(74) Representative: Sugrañes Patentes y Marcas
(86) International application number: PCT/ES2017/070279
(87) International publication number: WO 2017/191351

(57) **Abstract**

The present invention relates to delayed-release methylphenidate tablets characterized in that they are provided with a release-controlling layer comprising a combination of an enteric polymer and a hydrophobic agent. These tablets have a release profile characterized in that the drug does not substantially start to release until approximately three hours after administration, completing it after approximately four hours after administration. This profile allows for the preparation of a modified-release dosage form of methylphenidate, combining the delayed-release tablet with an additional fraction of immediate-release methylphenidate. The dosage form thus obtained is suitable for treating attention deficit hyperactivity disorder (ADHD).

## Description

### Application field

The present invention relates to new dosage forms of modified-release methylphenidate.

### State of the art

Attention deficit hyperactivity disorder (ADHD) is a behavior disorder that mainly manifests itself during childhood and adolescence, the main symptoms of which are hyperactivity, distraction, difficulty in paying attention and impulsive behavior, all of which have a negative impact on school performance.

ADHD has a high incidence among children and adolescents, and a recent study places the incidence of this disorder in around 5.5% of children and/or young people between the ages of 5 and 17 worldwide (Erskine et al., The global coverage of prevalence data for mental disorders in children and adolescents, 2016, Epidemiol. Psychiatr. Sci., doi:10.1017/S2045796015001158).

Methylphenidate is currently the first-choice drug for the treatment of ADHD. It is a mild stimulant for the central nervous system, and mainly acts by blocking the reuptake of noradrenaline and dopamine in the presynapic neuron, which causes an increase in the release of said monoamines to the extraneuronal space.

One of the main disadvantages of the therapeutic use of methylphenidate for the treatment of ADHD involves the short half-life thereof, which oscillates between 2 and 4 hours, and in children, it is estimated that it is approximately 2.5 hours, which presents difficulties in obtaining a correct dosage. Thus, since the duration of the therapeutic effect thereof is shorter than the length of the school day, it is usually necessary to administer repeated dosages of the drug, or use modified-release forms.

Numerous studies prove that the different technologies used to achieve the extended release of methylphenidate usually lead to different pharmacokinetic profiles and that, in turn, has a high incidence on the greater or lesser effectiveness of the drug and the greater or lesser incidence of the secondary effects thereof, as described, for example, in the article Maldonado R., Comparison of the pharmacokinetics and clinical efficacy of the new extended-release formulations of methylphenidate, Expert Opin. Drug Metab. Toxicol., 2013, 9 (8), p1001-14. The authors of this article conclude that the different modified-release formulations available on the market have expanded the range of therapeutic options available for doctors when treating the symptoms of ADHD and they should provide greater therapeutic flexibility and optimization for treating this disorder.

This need to increase the number of therapeutic options for treating ADHD in order to make it easier to fulfill the relevant medical needs, along with the convenience of optimizing the compositions from a galenic, organoleptic and/or manufacturing point of view, for example, is reflected in the high number of documents existing in the state of the art that refer to the different modified-release methylphenidate formulations.

The compositions described in the state of the art use different strategies in the formulation to obtain specific methylphenidate release profiles.

For example, the patent application WO-A-99/62496 describes a dosage form of methylphenidate characterized by an increasing release rate of the drug during a certain period of time, thanks to the use of osmotic-type tablets comprising a core with two drug layers that are consecutively released thanks to a third push layer that contains an expandable polymer. The tablets described are surrounded by a semi-permeable membrane that allows the fluid to enter from the outside and it is provided with an outlet hole suitable for the increasing release of the drug.

Furthermore, the patent application WO-A-00/35426 describes pellet-type multiparticle methylphenidate compositions, in which an inert core is coated with a drug layer, upon which a first coating formed by a hydrophobic polymer to provide sustained release is added, and a second coating made up of an enteric polymer is applied on top of this, with the purpose of delaying the release, preferably with a curing step after applying each of the polymer layers and with a final external drug layer for the immediate release thereof. The in vitro dissolution profile of said composition is characterized by an initial quick-release phase of the immediate-release first fraction, which is completely dissolved in less than half an hour, followed by a period with practically no drug release, until it passes to the conditions of the intestinal fluid, once the first two hours have passed, at which time the release of the second fraction of methylphenidate begins, which occurs continuously for up to 12 hours.

The patent application WO-A-2007/133583 describes sustained dosage forms of methylphenidate that provide constant drug release, of zero order, during at least four hours. The compositions described consist of a matrix tablet, the core of which contains the active ingredient along with a hydrophobic material, preferably ethylcellulose and/or glyceryl behenate, and optionally a release modifier, such as a high or low viscosity hydroxypropylcellulose. The tablet is covered by a release-modifying layer that contains a hydrophobic polymer, for example, ethylcellulose, and a pore-former hydrophilic agent, such as hypromellose.

Some of the compositions described in the state of the art provide a pulsatile-type release, similar to that which would be obtained with two or more immediate-release dosages. For this, the compositions described comprise one part of the dosage in the form of immediate release, and one or more subsequent dosages that have delayed-release via the use of suitable polymer coatings, typically enteric polymers.

As such, for example, the patent application US-A-2004/0156896 describes a pulsatile-type dosage form of methylphenidate that intends to reproduce the effect of multiple dosages with only one administration. Said dosage form consists of a tablet with a conventional core that contains methylphenidate, coated with a controlled-release layer based on an enteric polymer. A final layer of methylphenidate is placed on said enteric layer for its immediate release.

The patent application WO-A-99/03471 describes pulsatile-release modified dosage forms of methylphenidate that are based on the presence of different particle populations. Thus, the compositions described have multiple particles, combined with a particle population that immediately releases the drug and consists of inert cores coated with the drug, along with another delayed-release particle population that also contains an outer layer specifically made up of an ammonium methacrylate polymer that is responsible for said pulsatile release.

Despite the diverse solutions proposed in the state of the art, there is still the need to provide new modified dosage forms of methylphenidate in order to have new alternatives that effectively treat ADHD and thus meet the medical needs thereof.

### Object of the invention

The object of the present invention is a delayed-release methylphenidate tablet.

Another aspect of the present invention is a dosage form of methylphenidate that comprises said tablet.

Another aspect of the present invention relates to the use of said dosage form to treat attention deficit hyperactivity disorder (ADHD).

### Description of the drawings

Figure 1

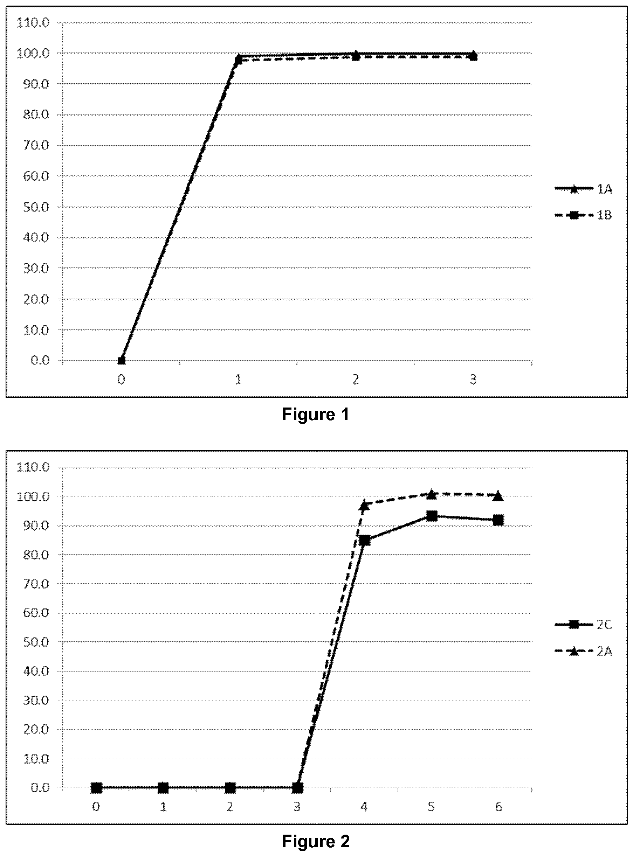

   Figure 1 shows the results of an in vitro dissolution trial, as described in Example 4, in which the quantity of dissolved methylphenidate is measured using the non-coated tablet cores (immediate-release tablets 1A and 1B, Example 1), according to a method that replicates the physiological conditions that consist in introducing the tablet into 500 ml of a 0.01N hydrochloric acid solution during 2 hours, at 37°C, and subsequently transferring the tablet to 500ml of a phosphate buffer solution of pH 6.8, at 37°C during 4 hours. The x-axis represents the time elapsed since the start of the trial (in hours), while the y-axis represents the percentage of methylphenidate dissolved, determined at intervals of 1 hour over a period of 3 hours.
Figure 2
   Figure 2 shows the results of an in vitro dissolution trial, as described in Example 4, in which the quantity of dissolved methylphenidate is measured using the delayed-release tablets of the invention (tablets 2A and 2C, Example 2), according to a method that replicates the physiological conditions that consist in introducing the tablet into 500 ml of a 0.01N hydrochloric acid solution during 2 hours, at 37°C, and subsequently transferring the tablet to 500ml of a phosphate buffer solution of pH 6.8, at 37°C during 4 hours. The x-axis represents the time elapsed since the start of the trial (in hours), while the y-axis represents the percentage of methylphenidate dissolved, determined at intervals of 1 hour over a period of 6 hours.
Figure 3

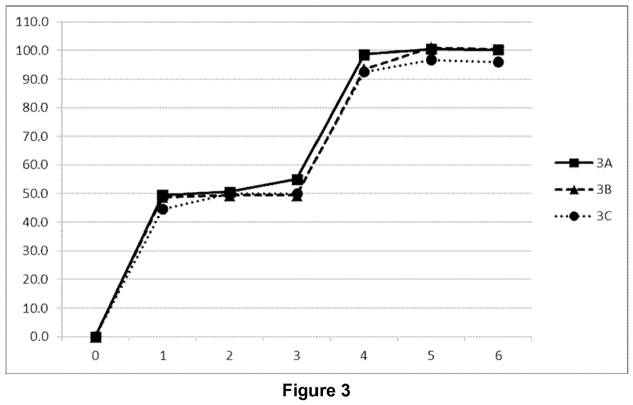

   Figure 3 shows the results of an in vitro dissolution trial, as described in Example 4, in which the quantity of dissolved methylphenidate is measured using the modified-release dosage form according to the invention (capsules 3A, 3B and 3C, Example 3), according to a method that replicates the physiological conditions that consist in introducing each capsule into 500 ml of a 0.01N hydrochloric acid solution during 2 hours, at 37°C, and subsequently transferring the capsule to 500ml of a phosphate buffer solution of pH 6.8, at 37°C during 4 hours. The x-axis represents the time elapsed since the start of the trial (in hours), while the y-axis represents the percentage of methylphenidate dissolved, determined at intervals of 1 hour over a period of 6 hours.

### Detailed description of the invention

The object of the present invention is a delayed-release methylphenidate tablet comprising:
a) a core comprising methylphenidate or a pharmaceutically acceptable salt thereof, and
b) a release-modifying layer;
characterized in that the release-modifying layer (b) comprises the combination of an enteric polymer and a hydrophobic agent.

The authors of the present invention have developed a delayed-release methylphenidate tablet in which, surprisingly, the combination of an enteric polymer and a hydrophobic agent in the controlling layer provides a characteristic methylphenidate release profile according to which the drug is not substantially released until after approximately 1 hour has passed in intestinal pH conditions, that is, is approximately 3 hours after administration; once the release begins, it occurs rapidly and completely. This release profile makes it possible to prepare a pulsatile dosage form combining an initial immediate-release dosage of methylphenidate, along with a second dosage contained in one or more said tablets, for its delayed release. The release profile achieved with said dosage form makes it particularly convenient for treating ADHD.

Throughout the present invention, unless the contrary is expressly indicated, all percentages and proportions always refer to the weight of the components.

In the context of the present invention, the term "approximately" is understood to refer to a specific value that indicates that some variation of said value is allowed, generally +/- 10%, preferably +/- 5%.

Throughout the present description as well as the claims, the expressions in singular preceded by the articles "a/an" or "the" are understood to also broadly include the reference to the plural, unless the context clearly indicates the contrary.

### Tablet core

The tablet core contains the active ingredient, methylphenidate.

Methylphenidate is the ICD that corresponds to methyl 2-phenyl-2-(2-piperidyl)acetate. The methylphenidate molecule has two chiral centers, which has four stereoisomers in the form of two pairs of enantiomers, which are usually called d-threo, l-threo, d-erythro and l-erythro. Methylphenidate generally refers to the racemic form threo, in other words, dl-threo-methyl 2-phenyl-2-(2-piperidyl)acetate.

In the scope of the present invention, the name methylphenidate broadly includes any of the said four forms, d-threo, l-threo, d-erythro and l-erythro, and the mixtures thereof, in any proportion.

Preferably, a mixture of the pair of threo enantiomers are used, in other words, a mixture of d- and l-threo methylphenidate, more preferably a racemic mixture, in other words, wherein both enantiomers have a 1:1 ratio, that is, dl-threo-methyl 2-phenyl-2-(2-piperidyl)acetate.

The methylphenidate d-threo- enantiomer, substantially free of other forms, can be used. This form is called dexmethylphenidate.

Methylphenidate can be used as a pharmaceutically acceptable salt, as well as possible hydrated or solvated forms. For example, hydrochloride, sulfate, tosilate, mesilate, maleate, tartrate, citrate or acetate salt can be used. Preferably, methylphenidate hydrochloride is used.

Methylphenidate hydrochloride is the most frequently used salt in the methylphenidate specialties that are commercially available. Throughout the present invention, the quantities and proportions described for methylphenidate always refer to the equivalent quantity of its hydrochloride salt.

The tablet core that contains methylphenidate is formulated as a conventional tablet. Typically, the tablets can be prepared either with methods that include previous granulation before compressing them, either by wet or dry granulation, or they can be prepared by direct compression of the components, in all cases following methods that are well-known by a person skilled in the art, as described in renowned pharmaceutical technology manuals, such as for example, the book Remington The Science and Practice of Pharmacy, 21st edition, Lippincott, Williams & Wilkins, Philadelphia, 2005 [ISBN: 0-683-306472], or the book Aulton's Pharmaceutics The Design and Manufacture of Medicines, 4th Edition, Churchill Livingstone Elsevier, 2013, [ISBN 978-0-7020-4290-4].

To prepare the tablet core, along with the active ingredient, at least one pharmaceutically acceptable excipient is typically used.

Among the pharmaceutically acceptable excipients that are normally used to prepare the tablet core of the invention, there are diluting agents, such as starch, pregelatinized starch, calcium carbonate, microcrystalline cellulose, powdered cellulose, cellulose acetate, kaolin, anhydrous lactose, lactose monohydrate, sodium chloride, calcium phosphate, anhydrous calcium phosphate hydrogen, dihydrate calcium phosphate hydrogen, calcium sulfate, magnesium carbonate, magnesium oxide, glyceryl palmitostearate, dextrates, dextrose, fructose, lactitol, mannitol, maltitol, maltodextrins, maltose, sucrose, sorbitol, among others, and the mixtures thereof. Generally, the diluting agent is in a proportion comprised between 50% and 95%, preferably comprised between 70% and 90%, and more preferably comprised between 80% and 85%, referring to the total weight of the tablet core.

Disintegrant agents can optionally be added, such as alginic acid, sodium alginate, starch, pregelatinized starch, calcium or sodium carboxymethyl cellulose, sodium croscarmellose, crospovidone, sodium starch glycolate, hydroxypropyl cellulose with a low degree of substitution, or sodium salt of the polymer of methacrylic acid with divinylbenzene (Amberlite®IRP-88), among others, and the mixtures thereof. By using a disintegrant, it typically adds a proportion comprised between 1% and 20%, preferably between 5% and 10%, referring to the total weight of the tablet core.

Sliding agents can also be optionally added, such as colloidal silica, magnesium stearate, talc, tribasic calcium phosphate, magnesium or calcium silicate, or magnesium trisilicate, among others, and the mixtures thereof. By using a sliding agent, it typically adds a proportion comprised between 0.1% and 5%, preferably between 0.2% and 2%, and more preferably between 0.5% and 1.0%, referring to the total weight of the tablet core.

Eventually, agglutinant agents can be used, such as hydroxypropylmethylcellulose, starch, dextrin, dextrose, polydextrose, maltodextrin, sucrose or povidone, among others, and the mixtures thereof. By using an agglutinant agent, it is generally used in a proportion comprised between 1% and 50%, preferably between 3% and 40%, and more preferably between 5% and 30%, referring to the total weight of the tablet core.

Lubricant agents are also typically used to favor the compression process, for example, stearic acid, magnesium stearate, calcium stearate, sodium stearate, glyceryl palmitostearate, poloxamers, magnesium oxide, sodium benzoate, colloidal silica, sodium lauryl sulphate, magnesium laurylsulfate, sodium stearyl fumarate, sodium oleate, hydrogenated castor oil, talc or glycerin behenate, among others, and the mixtures thereof. By using a lubricant agent, it is typically added in a proportion comprised between 0.1% and 5%, preferably between 0.2% and 2%, and more preferably between 0.5% and 1.0%, referring to the total weight of the tablet core.

The percentages of the different excipients are indicative and a person skilled in the art will know how to choose the appropriate excipients and their proportions, according to the method of preparation used, the appropriate proportion of the drug in the tablet, and/or the required properties thereof, for example, the size, appearance, stability and/or hardness. It is understood that the percentages of the excipient or excipients used, along with the percentage of the active ingredient add up to 100% of the tablet core.

The physical and chemical characteristics of the excipients, as well as the name of the commercial products under which they are sold, can be found in the book R.C. Rowe et al., Handbook of Pharmaceutical Excipients, 4th edition, Pharmaceutical Press, London, 2003 [ISBN: 0-85369-472-9].

The tablets can be prepared in different shapes and sizes, based on the punch and matrix used in the compression process, as is already well-known in the state of the art, and the shape that is considered to be the most convenient in each case can be chosen, for example, based on the different methylphenidate dosages. Likewise, once said tablets, which are the delayed-release tablets of the invention, are coated, they can be used to prepare modified-release dosage forms, as shall be described further on, for example, incorporating them in hard capsules. Another factor that can be taken into account when choosing the most appropriate shape and size of the tablets is that they can be easily stored within the capsules. For example, in a preferred embodiment, appropriate punches and matrices are chosen to prepare circular tablets, with a diameter comprised between 3 and 6 mm, preferably comprised between 4 and 5 mm.

In general, the amount of methylphenidate in the tablet core is comprised between 5% and 50%, preferably between 8% and 30%, more preferably between 12% and 20%, and even more preferably between 15% and 18%, wherein the percentages refer to the weight of methylphenidate with respect to the total weight of the tablet core, and wherein the quantity of methylphenidate is expressed as the equivalent quantity of methylphenidate hydrochloride.

In a preferred embodiment of the invention, the tablet core is prepared by direct compression.

In a preferred embodiment of the invention, the tablet core comprises:
- methylphenidate, in a proportion comprised between 5% and 50%, preferably between 8% and 30%, more preferably between 12% and 20%, and even more preferably between 15% and 18%;
- diluting agent, in a proportion comprised between 50% and 95%, preferably between 70% and 90%, and more preferably between 80% and 85%;
- lubricant, in a proportion comprised between 0.1% and 5%, preferably between 0.2% and 2%, and more preferably between 0.5% and 1.0%;
wherein the percentages refer to the weight of each component with respect to the total weight of the tablet core, wherein the quantity of methylphenidate is expressed as an equivalent quantity of methylphenidate hydrochloride.

In a preferred embodiment, the tablet core essentially consists of:
- methylphenidate, in a proportion comprised between 5% and 50%, preferably between 8% and 30%, more preferably between 12% and 20%, and even more preferably between 15% and 18%;
- a diluting agent, in a proportion comprised between 50% and 95%, preferably between 70% and 90%, and more preferably between 80% and 85%;
- lubricant, in a proportion comprised between 0.1% and 5%, preferably between 0.2% and 2%, and more preferably between 0.5% and 1.0%;
wherein the percentages refer to the weight of each component with respect to the total weight of the tablet core, and wherein the quantity of methylphenidate is expressed as the equivalent quantity of methylphenidate hydrochloride, and wherein the sum of the percentages is equal to 100%.

The diluting agent is preferably chosen from starch, microcrystalline cellulose, powdered cellulose, anhydrous lactose, lactose monohydrate, mannitol, maltitol, sucrose, sorbitol, and the mixtures thereof; more preferably it is chosen from among anhydrous lactose, microcrystalline cellulose and the mixtures thereof.

In a particularly preferred embodiment, a mixture of anhydrous lactose and microcrystalline cellulose is used, preferably in a ratio of microcrystalline cellulose:anhydrous lactose comprised between 0.5:1 and 2:1, more preferably comprised between 1:1 and 1.5:1.

In a particularly preferred embodiment, the tablet core essentially consists of:
- methylphenidate, in a proportion comprised between 5% and 50%, preferably between 8% and 30%, more preferably between 12% and 20%, and even more preferably between 15% and 18%;
- microcrystalline cellulose, in a proportion comprised between 30% and 55%, preferably between 35% and 50%, more preferably comprised between 40% and 45%;
- anhydrous lactose, in a proportion comprised between 20% and 45%, preferably comprised between 25% and 45%, and more preferably comprised between 35% and 40%; and
- lubricant, in a proportion comprised between 0.1% and 5%, preferably between 0.2% and 2%, and more preferably between 0.5% and 1.0%;
wherein the percentages refer to the weight of each component with respect to the total weight of the tablet core, and wherein the quantity of methylphenidate is expressed as an equivalent quantity of methylphenidate hydrochloride, and wherein the sum of the percentages is equal to 100%.

The delayed-release tablet core, before applying the release-modifying layer to it, is in itself a tablet for the immediate release of methylphenidate. This core was subject to an in vitro dissolution test in which the physiological conditions were simulated (the first 2 hours in a 0.01N hydrochloric acid solution, and the subsequent hours in a phosphate buffer solution of pH 6.8), as described in Example 4, and it was verified that one hour after the start of the trial, all methylphenidate had been substantially dissolved (Figure 1).

### Release-modifying layer

The delayed-release tablet of the present invention is made up of the tablet core, as previously described, and a release-modifying layer that coats it.

The release-modifying layer is characterized in that it comprises a combination of two release-controlling agents: an enteric polymer and a hydrophobic agent, and said combination is what gives the tablets of the invention their characteristic release profile.

The enteric profile refers, as is well known in the art, to a coating agent that remains substantially intact in the acid pH conditions of the stomach, but which dissolves in the pH conditions of the intestine.

The enteric polymer is preferably chosen from among the group formed by a copolymer of methacrylic acid with methyl methacrylate, a copolymer of methyl acrylate with methyl methacrylate and methacrylic acid, cellulose acetate phthalate, hydroxypropyl methylcellulose acetate succinate, hydroxypropyl methylcellulose phthalate, polyvinyl acetate phthalate, cellulose acetate trimellitate, and the mixtures thereof.

In a preferred embodiment of the invention, the enteric polymer is chosen from among the group formed by hydroxypropyl methylcellulose acetate succinate, cellulose acetate phthalate, polyvinyl acetate phthalate, and the mixtures thereof.

In a particularly preferred embodiment, the enteric polymer is hydroxypropyl methylcellulose acetate succinate (HPMCAS, in short).

The hydrophobic agent name refers to a coating agent that provides sustained release, independent from pH, which is also well-known in the art, and said hydrophobic agent can be either a fatty substance, preferably a fatty acid triglyceride or a fatty alcohol ester with a fatty acid, or a hydrophobic polymer.

Among the fatty substances suitable for use as a hydrophobic agent in the present invention, there are, for example, hydrogenated vegetable oils, such as hydrogenated castor oil, among others, white beeswax (cera alba) or yellow beeswax (cera flava).

Among the hydrophobic polymers suitable for use as a hydrophobic agent in the present invention, there are, for example, ethylcellulose, methylcellulose, hydroxypropyl cellulose, a copolymer of ethyl acrylate with methyl methacrylate, or a copolymer of ethyl acrylate with methyl methacrylate and trimethylammonium ethyl methacrylate.

In an embodiment of the invention, the hydrophobic agent is chosen from among the group formed by ethylcellulose, methylcellulose, hydroxypropyl cellulose, hydrogenated castor oil, white beeswax (cera alba), yellow beeswax (cera flava), a copolymer of ethyl acrylate with methyl methacrylate, a copolymer of ethyl acrylate with methyl methacrylate and trimethylammonium ethyl methacrylate, and the mixtures thereof.

In a preferred embodiment of the invention, the hydrophobic agent is a hydrophobic polymer chosen from among the group formed by ethylcellulose, methylcellulose, hydroxypropyl cellulose, a copolymer of ethyl acrylate with methyl methacrylate, a copolymer of ethyl acrylate with methyl methacrylate and trimethylammonium ethyl methacrylate, and the mixtures thereof.

In another preferred embodiment of the invention, the hydrophobic agent is chosen from among the group formed by ethylcellulose, a copolymer of ethyl acrylate with methyl methacrylate, hydrogenated castor oil, and the mixtures thereof.

In a particularly preferred embodiment, the hydrophobic agent is ethylcellulose.

Generally, the weight ratio of enteric polymer:hydrophobic agent is comprised between 2:1 and 1:1, preferably comprised between 1.6:1 and 1.1:1, more preferably comprised between 1.4:1 and 1.2:1, and even more preferably is approximately 1.3:1.

The release-modifying layer contains the combination of enteric polymer and hydrophobic agent, and possibly also contains one or more pharmaceutically acceptable excipients.

The modifying layer also preferably includes a plasticizing agent to improve the flexibility of the polymers included in the coating and thus provide a more homogeneous tablet coating. The plasticizing agent can be chosen, for example, from among the following: citric acid esters, such as acetyl tributyl citrate, acetyl triethyl citrate, tributyl citrate or triethyl citrate; phthalic acid esters, such as dibutyl phthalate, diethyl phthalate or dimethyl phthalate; dibutyl sebacate, benzil benzoate, glycerin, glycerol monostearate, cetyl alcohol, triacetin, propylene glycol, polyethylene glycol, and the mixtures thereof. Preferably, the plasticizing agent is chosen from among the citric acid esters, cetyl alcohol, and the mixtures thereof.

In a preferred embodiment of the invention, the release-modifying layer comprises a plasticizing agent consisting of the mixture of triethyl citrate and cetyl alcohol, wherein the ratio of triethyl citrate:cetyl alcohol is preferably comprised between 10:1 and 4:1, more preferably comprised between 8:1 and 6:1, and even more preferably the triethyl citrate:cetyl alcohol ratio is approximately 7:1.

The total proportion of the plasticizing agent in the release-modifying layer is generally comprised between 10% and 30%, preferably comprised between 15% and 25%, referring to the total weight of said layer.

The release-modifying layer can also include a sliding agent such as talc, colloidal silica, magnesium or calcium silicate, among others, and the mixtures thereof. The sliding agent is preferably talc.

The total proportion of the sliding agent in the release-modifying layer is generally comprised between 5% and 20%, preferably comprised between 10% and 15%, referring to the total weight of said layer.

The release-modifying layer can include other auxiliary excipients, such as surfactants or colorants, among others.

For example, a surfactant can be used to improve the wettability of the coating agents and thus facilitate their incorporation into the aqueous suspensions usually used to apply the release-modifying layer. Non-ionic or anionic surfactants can be used, for example, sorbitan esters, poloxamers, sodium lauryl sulphate, docusate sodium or isopropylamine dodecylbenzenesulphonate. Sodium lauryl sulphate is preferably used.

A colorant can be used in the release-modifying layer to make it easier to visually distinguish the delayed-release tablets of the invention from other tablets present in the same dosage form, for example, immediate-release tablets. The colorant can be chosen, for example, from lactoflavin phosphate, sodium chlorophyllin, red beet, and enocianina, among others.

The sum of the enteric polymer and the hydrophobic agent is usually made up of between 55% and 85% of the total weight of the release-modifying layer, preferably between 60% and 75%, more preferably between 62% and 70%, and even more preferably between 64% and 66%.

In a preferred embodiment of the invention, the release-modifying layer essentially consists of:
- between 33% and 40%, preferably between 36% and 38% of enteric polymer;
- between 25% and 33%, preferably between 27% and 30% of hydrophobic agent;
- between 15% and 25%, preferably between 18% and 23% of plasticizing agent;
- between 8% and 14%, preferably between 10% and 12% of sliding agent; and
- between 0% and 4% of other excipients;
wherein the percentages refer to the weight of each component with respect to the total weight of the release-modifying layer;
wherein the enteric polymer is preferably hydroxypropyl methylcellulose acetate succinate and the hydrophobic agent is preferably ethylcellulose;
wherein the sliding agent is preferably talc;
wherein the other excipients are preferably chosen from surfactants, colorants, and the mixtures thereof; more preferably wherein the surfactant is sodium lauryl sulphate; and
wherein the sum of percentages makes up 100%.

In an even more preferred embodiment of the invention, the release-modifying layer essentially consists of:
- between 33% and 40%, preferably between 36% and 38% of hydroxypropyl methylcellulose acetate succinate;
- between 25% and 33%, preferably between 27% and 30% of ethylcellulose;
- between 15% and 20%, preferably between 16% and 19% of triethyl citrate;
- between 1.5% and 3.5%, preferably between 2% and 3% of cetyl alcohol;
- between 8% and 14%, preferably between 10% and 12% of talc; and
- between 0% and 4% of other excipients;
wherein the percentages refer to the weight of each component with respect to the total weight of the release-modifying layer;
wherein the other excipients are preferably chosen from surfactants, colorants, and the mixtures thereof; more preferably wherein the surfactant is sodium lauryl sulphate; and
wherein the sum of percentages makes up 100%.

A sufficient quantity of the release-modifying layer is applied to the tablet core so as to release the active ingredient according to the required profile.

Generally, the release-modifying layer is between 15% and 23%, preferably between 16% and 22%, and more preferably between 16.5% and 21% of the total weight of the tablet.

The tablet coating is made by following a normal method of the state of the art, usually preparing an aqueous suspension of the enteric polymer and the hydrophobic agent, eventually with the rest of the excipients present in the release-modifying layer, spraying said suspension onto the tablet cores and removing the water, by drying for example, leaving a deposit of the coating material on the cores.

To carry out this process, the usual methods and equipment, well-known by a person skilled in the art, can be used. For example, a coating drum equipped with an injection system of the coating suspension and also equipped with a local vacuum system can be used; or equipment that operates according to the principle of the fluid bed can be used.

The coated tablets obtained can be subject to a curing process through treatment at temperatures usually comprised between 55°C and 75°C, to favor the coalescense of the coating materials.

The aqueous suspension that is applied on the cores contains the enteric polymer, the hydrophobic agent and, eventually, the plasticizing agent, sliding agent, surfactant and/or colorant. The prepared suspension generally contains between 75% and 93% of water.

### Dosage form

The delayed-release tablet of the present invention was subjected to an in vitro dissolution test in which pH gastric conditions were simulated during the first 2 hours (0.01N hydrochloric acid solution at 37°C) and intestinal conditions during the next 4 hours (phosphate buffer solution of pH 6.8 at 37°C), as described in Example 4.

It was observed, surprisingly, that the drug was not substantially released during the first 3 hours of the trial, and at this moment, the methylphenidate was completely and quickly released, substantially producing the total dissolution of the drug in approximately 1 hour, which provided a delayed-release "pulse" of the product 3 hours after ingestion (Figure 2).

This release profile is particularly convenient for preparing dosage forms of methylphenidate, combining this delayed-release tablet along with an initial immediate-release dosage. This combination provides a release profile characterized by an initial "pulse", due to the first immediate dosage, and a second "pulse", due to the delayed-release tablet, as shown in Example 4 (Figure 3). The temporary location of the second pulse provided by the tablet of the present invention, started approximately 3 hours after administration and practically completed at the end of the fourth hour, is very convenient for providing a prolonged and reliable effect of the therapeutic action of the drug, suitable for lasting throughout the entire school day.

Therefore, another aspect of the present invention is a dosage form of methylphenidate that comprises said delayed-release tablet.

In a preferred embodiment, the dosage form comprises the tablet of the invention and an additional immediate-release dosage of methylphenidate, to thus obtain a modified-release dosage form of methylphenidate.

In the framework of the present invention, an "immediate release" methylphenidate tablet, or a fraction of methylphenidate, or a dosage of methylphenidate, refers to the fact that the release or dissolution of the drug begins immediately or a few minutes after administration, such that at least 80% of the drug, preferably at least 90% of the drug, dissolves during the first hour, as can be determined in an in vitro dissolution trial that replicates the gastric pH conditions (0.01N hydrochloric acid solution).

In the framework of the present invention, a delayed-release methylphenidate tablet, or a fraction of methylphenidate, or a dosage of methylphenidate, refers to the fact that the release or dissolution of the drug does not substantially begin until after a certain period of time after administration, at least 1 hour, preferably at least 2 hours, and more preferably of approximately 3 hours, as can be determined in an in vitro dissolution trial that replicates the physiological conditions (the first 2 hours in 0.01N solution of hydrochloric acid and the subsequent hours in a phosphate buffer solution of pH 6.8).

In the framework of the present invention, a modified- or extended-release dosage form is understood to be any dosage form that is not immediately released, and it can include different prolonged-release forms of the drug throughout time. Particularly, a modified dosage form includes pulsatile release of the drug, with a fraction thereof that is released immediately and another fraction that is delay-released.

In a preferred embodiment, the dosage form of the present invention usually contains a complete daily dosage of methylphenidate, which is released over an extended period, making it possible to maintain the therapeutic effect throughout the whole school day without needing to administer additional dosages of the drug.

The complete daily dosage of methylphenidate contained in the dosage form can vary based on the patient and their therapeutic needs, for example, according to age and the severity of the disorder, depending on the therapeutic dosages generally accepted for this active ingredient, which are well-known by a person skilled in the art. Generally, it is considered that the daily dosage of methylphenidate should not exceed 60 mg, such that the total dosage of methylphenidate contained in the dosage form of the invention is preferably comprised between 5 mg and 60 mg, more preferably comprised between 10 mg and 40 mg of methylphenidate, expressed as an equivalent quantity of methylphenidate hydrochloride, although these dosages can be greater if so required by the therapeutic guidelines desired.

Therefore, each dosage form contains a complete daily dosage of methylphenidate, typically distributed between an immediate-release fraction and a delayed-release fraction.

The proportion of immediate-release and delayed-release methylphenidate can vary, based on the specific release profile required for said dosage form. Generally, the proportion of immediate-release methylphenidate is comprised between 30% and 60%, preferably between 40% and 55%, and more preferably is approximately 50% of the total dosage of methylphenidate; while the proportion of delayed-release methylphenidate is generally comprised between 40% and 70%, preferably between 45% and 60%, and more preferably is approximately 50% of the total dosage of methylphenidate.

The dosage form of methylphenidate of the present invention is preferably in a hard capsule form, typically of gelatin or hypromellose, which contains the fraction of immediate-release methylphenidate and the delayed-release tablet.

The fraction of immediate-release methylphenidate contained in said dosage form can have different forms, for example, it can be in powder form or granules, or in the form of immediate-release tablets, or in the form of immediate-release pellets, for example, inert cores coated in the active ingredient, or matrix pellets, which are well-known by a person skilled in the art.

In a preferred embodiment, the fraction of immediate-release methylphenidate contained in the dosage form of the invention is in the form of immediate-release tablets, and therefore, in this case, the dosage form comprises a delayed-release methylphenidate tablet according to the present invention, and an immediate-release methylphenidate tablet.

In a particularly preferred embodiment, the immediate-release tablet is a tablet core of the invention, devoid of the release-modifying layer.

Eventually, each one of the immediate-release and delayed-release fractions can be distributed among more than one tablet of each type.

In an embodiment of the invention, the dosage form comprises one or more immediate-release tablets that make up the immediate-release fraction and one or more delayed-release tablets that make up the delayed-release fraction.

In a preferred embodiment of the invention, the dosage form comprises between 1 and 6, preferably between 1 and 4, delayed-release tablets and between 1 and 6, preferably between 1 and 4, immediate-release tablets.

In an embodiment of the invention, all tablets comprised in both the immediate-release and delayed-release dosage form substantially contain the same quantity of methylphenidate. Preferably, both types of tablets are combined in the same number in the dosage form, until the total dosage required is completed, such that the proportions of immediate-release and delayed-release methylphenidate are substantially equal, that is, approximately 50% of each type.

In alternative preferred embodiments of the invention, the dosage form is a hard capsule, typically of gelatin or hypromellose, which contains:
- 1 immediate-release tablet and 1 delayed-release tablet; or
- 2 immediate-release tablets and 2 delayed-release tablets; or
- 3 immediate-release tablets and 3 delayed-release tablet; or
- 4 immediate-release tablets and 4 delayed-release tablets; or
- 5 immediate-release tablets and 5 delayed-release tablet; or
- 6 immediate-release tablets and 6 delayed-release tablet;
wherein all tablets comprised in both the immediate-release and delayed-release dosage form, substantially contain the same quantity of methylphenidate, in other words, the methylphenidate contained in the immediate-release tablet(s) makes up approximately 50% of the total dosage of methylphenidate, and the methylphenidate contained in the delayed-release tablet(s) approximately makes up the remaining 50% of the total dosage of methylphenidate.

In a preferred embodiment of the invention, the methylphenidate dosage of each one of the tablets contained in the dosage form, both of delayed-release and immediate-release, is comprised between 3 mg and 40 mg, preferably comprised between 5 mg and 30 mg, and the dosage is more preferably chosen from: 5 mg, 10 mg, 15 mg, 20 mg, 25 mg and 30 mg approximately, expressed as an equivalent quantity of methylphenidate hydrochloride.

In alternative preferred embodiments of the invention, the dosage form contains:
- 1 immediate-release tablet and 1 delayed-release tablet, wherein both tablets contain the same dosage of methylphenidate that is chosen from 5 mg, 10 mg, 15 mg, 20 mg, 25 mg and 30 mg, approximately, of methylphenidate;
- 2 immediate-release tablets and 2 delayed-release tablets, wherein all the tablets contain the same dosage of methylphenidate that is chosen from 5 mg, 10 mg and 15 mg, approximately, of methylphenidate;
- 3 immediate-release tablets and 3 delayed-release tablets, wherein all the tablets contain the same dosage of methylphenidate that is chosen from 5 mg and 10 mg, approximately, of methylphenidate;
- 4 immediate-release tablets and 4 delayed-release tablets, wherein each one of the tablets contain 5 mg, approximately, of methylphenidate;
- 5 immediate-release tablets and 5 delayed-release tablets, wherein each one of the tablets contain 5 mg, approximately, of methylphenidate;
- 6 immediate-release tablets and 6 delayed-release tablets, wherein each one of the tablets contain 5 mg, approximately, of methylphenidate;
wherein the quantities of methylphenidate are expressed as an equivalent quantity of methylphenidate hydrochloride.

In a particularly preferred embodiment of the invention, immediate-release and delayed-release tablets with a relatively low dosage of methylphenidate, of approximately 5 mg, are prepared, such that dosage forms with different total dosages of methylphenidate, including the correct number of tablets in the dosage form, can be prepared easily and in a versatile way.

Thus, in order to prepare a total dosage of 10 mg, 1 immediate-release tablet and 1 delayed-release tablet is introduced; to prepare a dosage of 20 mg, 2 tablets of each type are introduced; to prepare 30 mg tablets, 3 tablets of each type are introduced; to prepare a dosage of 40 mg, 4 tablets of each type are introduced; and so on.

This has the additional advantage of making it easier to manufacture the dosage form, since it requires the preparation of only one tablet of each type.

In an alternative embodiment of the invention, the combination of immediate-release and delayed-release tablets can be provided in alternative means, instead of being stored in a hard capsule, as previously described. For example, it can provide the required combination of both types of tablets to be ingested in each dosage, contained in a suitable single-dose container, for example, in sachets or in blister packs.

### Use of the dosage form of the invention

The dosage form of the present invention provides a release of methylphenidate in two "pulses", one immediate, during the first hour after administration, and the second that has a delayed start, approximately 3 hours after administration, as shown in Example 4 (Figure 3).

This profile is particularly appropriate for treating ADHD in children and young people since it can provide effective levels of the drug in the blood at the start of the school day, and be adequately extended to maintain a dilated therapeutic effect throughout the duration of said period.

Thus, another aspect of the present invention is the modified-release dosage form of the invention for its use in treating attention deficit hyperactivity disorder (ADHD).

In other words, another aspect of the present invention is the use of the modified-release dosage form of the invention to prepare a medicine to treat attention deficit hyperactivity disorder (ADHD).

In other words, another aspect of the present invention is a method for treating attention deficit hyperactivity disorder (ADHD) in a subject that needs it, comprising the administration of the modified-release dosage form of the invention.

In the framework of the present invention, the term treatment refers to the elimination, reduction, improvement or alleviation of the symptoms associated with attention deficit hyperactivity disorder (ADHD).

### Examples

### Example 1 Preparation of the tablet core (immediate-release tablet)

Tablets containing 5 mg of methylphenidate hydrochloride were prepared using the components and quantities that are listed in the following table.

| Example 1A | |
|---|---|
| Component | Weight (mg) |
| Methylphenidate HCl | 5.0 |
| Anhydrous lactose | 11.6 |
| Microcrystalline cellulose | 13.2 |
| Magnesium stearate | 0.2 |
| Total | 30.0 |

The different components are passed through a sieve and are mixed until a homogeneous mixture is obtained. The tablets are formed by direct compression of the mixture.

In the same way, tablets containing 20 mg of methylphenidate were prepared using the components that are indicated in the following table.

| Example 1B | |
|---|---|
| Component | Weight (mg) |
| Methylphenidate HCl | 20.0 |
| Anhydrous lactose | 46.4 |
| Microcrystalline cellulose | 52.8 |
| Magnesium stearate | 0.8 |
| Total | 120 |

### Example 2 Preparation of delayed-release tablets

To prepare the delayed-release tablets, an aqueous suspension was prepared using the components and the proportions indicated in the following table:

| Component | % (by weight) |
|---|---|
| Ethylcellulose | 4.2 |
| HPMCAS | 5.5 |
| Talc | 1.6 |
| Triethyl citrate | 2.8 |
| Cetyl alcohol | 0.4 |
| Sodium lauryl sulphate | 0.3 |
| Colorant | 0.1 |
| Purified water | 85.0 |

The cores prepared in Example 1A were coated using this suspension. The coated tablets obtained were cured by treatment at 70°C during 2 hours. The coated tablets weighed 36.0 mg (Coated tablets 2A). Following a similar method, using the same cores of Example 1A, coated tablets with total weight of 37.8 mg were prepared (Coated tablets 2B).

Similarly, the cores prepared in Example 1B were coated using the same suspension, and subsequently cured in the same way. The coated tablets weighed 152.4 mg (Coated tablets 2C).

The composition of the tablets obtained is shown in the following table:

| | Tablets | | |
|---|---|---|---|
| Component | 2A | 2B | 2C |
| *Core* | Weight (mg) | | |
| Methylphenidate HCl | 5.0 | 5.0 | 20.0 |
| Anhydrous lactose | 11.6 | 11.6 | 46.4 |
| Microcrystalline cellulose | 13.2 | 13.2 | 52.8 |
| Magnesium stearate | 0.2 | 0.2 | 0.8 |
| Core total | 30.0 | 30.0 | 120 |

| *Coating* | | | |
|---|---|---|---|
| HPMCAS | 2.2 | 2.8 | 11.9 |
| Ethylcellulose | 1.7 | 2.2 | 9.1 |
| Talc | 0.6 | 0.9 | 3.6 |
| Triethyl citrate | 1.1 | 1.4 | 6.0 |
| Sodium lauryl sulphate | 0.1 | 0.2 | 0.7 |
| Cetyl alcohol | 0.2 | 0.2 | 0.9 |
| Colorant | 0.1 | 0.1 | 0.2 |
| Coating total | 6.0 | 7.8 | 32.4 |
| Tablet total | 36.0 | 37.8 | 152.4 |

### Example 3 Preparation of modified-release dosage forms

Modified-release dosage forms were prepared introducing a combination of immediate-release tablets (Example 1) and delayed-release tablets (Example 2) into a hard gelatin capsule, as indicated in the following table:

| Example | Immediate-release | Delayed-release | Total dosage of Methylphenidate HCl |
|---|---|---|---|
| 3A | 4 tablets | 4 tablets | 40 mg |
| | Example 1A | Example 2A | |
| 3B | 4 tablets | 4 tablets | 40 mg |
| | Example 1A | Example 2B | |
| 3C | 1 tablet | 1 tablet | 40 mg |
| | Example 1B | Example 2C | |

In all cases, the capsules contained the same proportion of immediate-release drug and delayed-release drug, in other words, 50% each.

### Example 4 In vitro dissolution trials

An in vitro dissolution trial was carried out to study the methylphenidate release profile obtained with the delayed-release tablets of the invention and with the modified-release dosage forms prepared with them. The dissolution profile of the tablet core was also tested, which is in itself an immediate-release tablet.

To do so, a standard method that replicates the physiological conditions is followed, which consists of introducing a tablet or a capsule into 500 ml of a 0.01N hydrochloric acid solution during 2 hours, at 37°C, and subsequently transferring the tablet/capsule to 500ml of a phosphate buffer solution of pH 6.8 (prepared according to the specifications of the European Pharmacopoeia) at 37°C during 4 hours.

The quantity of dissolved methylphenidate was analyzed at intervals of 1h.

This dissolution trial was carried out with the tablets prepared in Example 1 (1A and 1B). The results are shown in Figure 1. The x-axis represents the time elapsed since the start of the trial (in hours), while the y-axis represents the percentage of methylphenidate dissolved. It can be observed that the methylphenidate dissolves quickly, since, after the first hour of the experiment, when making the first measurement, nearly all of the methylphenidate had already dissolved.

This dissolution trial also was carried out with the tablets prepared in Example 2 (2A and 2C). The results are shown in Figure 2. The x-axis represents the time elapsed since the start of the trial (in hours), while the y-axis represents the percentage of methylphenidate dissolved.

As shown in said figure, the release of methylphenidate does not occur until the third hour of the trial. At this time, the drug is quickly released.

Finally, this trial was also carried out for the capsules prepared in Example 2, which contained the combination of immediate-release and delayed-release tablets. The results are shown in Figure 3. The x-axis represents the time elapsed since the start of the trial (in hours), while the y-axis represents the percentage of methylphenidate dissolved.

As shown in said figure, all the tablets of Example 3 (3A, 3B and 3C) behaved in a practically identical way. The immediate-release fraction was quickly released in all cases, such that after the first hour, practically the whole amount thereof had already dissolved (50% of the total dosage). From then on, the drug was not substantially released until hour 3 of the trial, after 1 hour of being in pH 6.8 conditions, the time at which the second fraction of the drug was quickly released, such that after the fourth hour, the entire dosage of methylphenidate had already been practically released.

## Claims

1. A delayed-release tablet comprising:
(a) a core comprising methylphenidate or a pharmaceutically acceptable salt thereof, and
(b) a release-modifying layer;
**characterized in that** the release-modifying layer (b) comprises the combination of an enteric polymer and a hydrophobic agent.

2. The tablet according to claim 1, **characterized in that** the methylphenidate is in the form of hydrochloride salt.

3. The tablet according to any of the claims 1 or 2, **characterized in that** the tablet core comprises:
- methylphenidate, in a proportion comprised between 5% and 50%;
- diluting agent, in a proportion comprised between 50% and 95%;
- lubricant, in a proportion comprised between 0.1% and 5%;
wherein the percentages refer to the weight of each component with respect to the total weight of the tablet core, and wherein the quantity of methylphenidate is expressed as an equivalent quantity of methylphenidate hydrochloride.

4. The tablet according to any of the claims 1 to 3, **characterized in that** a mixture of anhydrous lactose and microcrystalline cellulose is used as a diluting agent.

5. The tablet according to any of the claims 1 to 4, **characterized in that** the tablet core essentially consists of:
- methylphenidate, in a proportion comprised between 5% and 50%;
- microcrystalline cellulose, in a proportion comprised between 30% and 55%;
- anhydrous lactose, in a proportion comprised between 20% and 45%; and
- lubricant, in a proportion comprised between 0.1% and 5%;
wherein the percentages refer to the weight of each component with respect to the total weight of the tablet core, and wherein the quantity of methylphenidate is expressed as an equivalent quantity of methylphenidate hydrochloride, and wherein the sum of the percentages is equal to 100%.

6. The tablet according to any of the claims 1 to 5, **characterized in that** the enteric polymer is chosen from among the group formed by a copolymer of methacrylic acid with methyl methacrylate, a copolymer of methyl acrylate with methyl methacrylate and methacrylic acid, cellulose acetate phthalate, hydroxypropyl methylcellulose acetate succinate, hydroxypropyl methylcellulose phthalate, polyvinyl acetate phthalate, cellulose acetate trimellitate, and the mixtures thereof.

7. The tablet according to claim 6, **characterized in that** the enteric polymer is hydroxypropyl methylcellulose acetate succinate.

8. The tablet according to any of the claims 1 to 7, **characterized in that** the hydrophobic agent is chosen from among the group formed by ethylcellulose, methylcellulose, hydroxypropyl cellulose, hydrogenated castor oil, white beeswax (cera alba), yellow beeswax (cera flava), a copolymer of ethyl acrylate with methyl methacrylate, a copolymer of ethyl acrylate with methyl methacrylate and trimethylammonium ethyl methacrylate, and the mixtures thereof.

9. The tablet according to claim 8, **characterized in that** the hydrophobic agent is ethylcellulose.

10. The tablet according to any of the claims 1 to 9, **characterized in that** the weight ratio of enteric polymer:hydrophobic agent is comprised between 2:1 and 1:1.

11. The tablet according to any of the claims 1 to 10, **characterized in that** the release-modifying layer essentially consists of:
- between 33% and 40% of enteric polymer;
- between 25% and 33% of hydrophobic agent;
- between 15% and 25% of plasticizing agent;
- between 8% and 14% of sliding agent; and
- between 0% and 4% of other excipients that are chosen from surfactants, colorants and the mixtures thereof;
wherein the percentages refer to the weight of each component with respect to the total weight of the release-modifying layer; and
wherein the sum of percentages makes up 100%.

12. The tablet according to any of the claims 1 to 11, **characterized in that** the release-modifying layer essentially consists of:
- between 33% and 40% of hydroxypropyl methylcellulose acetate succinate;
- between 25% and 33% of ethylcellulose;
- between 15% and 20% of triethyl citrate;
- between 1.5% and 3.5% of cetyl alcohol;
- between 8% and 14% of talc; and
- between 0% and 4% of other excipients that are chosen from surfactants, colorants and the mixtures thereof;
wherein the percentages refer to the weight of each component with respect to the total weight of the release-modifying layer; and
wherein the sum of percentages makes up 100%.

13. The tablet according to any of the claims 1 to 12, **characterized in that** the release-modifying layer is between 15% and 23% of the total weight of the tablet.

14. A dosage form that comprises a delayed-release tablet according to any of the claims 1 to 13.

15. The dosage form according to claim 14, **characterized in that** it further comprises an immediate-release methylphenidate tablet.

16. The dosage form according to claim 15, **characterized in that** the immediate-release methylphenidate tablet is the core of the delayed-release tablet as described in claims 3 to 5.

17. The dosage form according to any of the claims 15 or 16, **characterized in that** it comprises between 1 and 6 delayed-release tablets and between 1 and 6 immediate-release tablets.

18. The dosage form according to claim 17, **characterized in that** all the tablets substantially contain the same quantity of methylphenidate and that the dosage form contains the same number of immediate-dosage tablets as delayed-dosage tablets.

19. The dosage form according to any of the claims 17 or 18, **characterized in that** each tablet comprises a methylphenidate dosage of approximately 5 mg, expressed as an equivalent weight of methylphenidate hydrochloride.

20. The dosage form according to any of the claims 14 to 19, **characterized in that** the dosage form is in the form of a hard gelatin or hypromellose capsule.

21. A use of a dosage form according to any of the claims 14 to 20 to prepare a medicine to treat attention deficit hyperactivity disorder (ADHD).
